# EUROPEAN PATENT APPLICATION

(11) **EP 4 692 070 A1**
(43) Date of publication of application: **11.02.2026**
(21) Application number: 25716963.1
(22) Date of filing: 26.02.2025
(51) Int. Cl.: C07D 307/68, B01J 23/889, B01J 8/02

(54) **METHOD AND DEVICE FOR PREPARING FURANDICARBOXYLIC ACID**

(30) Priority: 11.06.2024 CN 202410747224
(71) Applicant: Hefei Leaf Biotech Co., Ltd., Hefei City Anhui 230088 (CN)
(72) Inventor: XU, Hai, Hefei, Anhui 230088 (CN); YU, Sanxi, Hefei, Anhui 230088 (CN); XU, Qiang, Hefei, Anhui 230088 (CN); LI, Xinglong, Hefei, Anhui 230088 (CN)
(74) Representative: Murgitroyd & Company
(86) International application number: PCT/CN2025/079285
(87) International publication number: WO 2025/256190

(57) **Abstract**

The present application discloses a method and apparatus for preparing furan dicarboxylic acid, more specifically, the method comprises: using 5-hydroxymethylfurfural as raw material, water as reaction solvent with added alkali to obtain a mixed reaction liquid, using a fixed bed as reactor, multi-metal LaₓCo_{y}MnV_{z} with different proportions as catalyst, at a certain temperature and oxygen pressure, flowing through the catalyst bed layer at a certain feed rate to obtain an aqueous solution containing furan dicarboxylate, precipitating furan dicarboxylic acid through acidification, and filtering to obtain the solid product. This method yields high product recovery, features a simple process route, and has potential industrial application prospects.

## Description

### TECHNICAL FIELD

The present application belongs to the technical field of furan dicarboxylic acid synthesis, particularly relating to a method and apparatus for preparing furan dicarboxylic acid.

### BACKGROUND

2,5-furan dicarboxylic acid (FDCA) is one of the twelve important platform molecules designated by the U.S. Department of Energy. The most important application of FDCA is as a polymer material monomer used to prepare polyesters, polyamides, polyurethanes, *etc.* Polyesters formed from FDCA have great potential to replace polyesters such as PET and PBT, offering advantages such as biodegradability and environmental friendliness. At the same time, FDCA as an important raw material also has significant applications in fields such as pharmaceuticals, pesticides, fire protection, and plasticizers.

5-hydroxymethylfurfural (abbreviated as HMF), HMF can be oxidatively converted into various important compounds, such as maleic anhydride, furan dialdehyde, furan dicarboxylic acid, *etc.* Currently, there are many studies on methods for preparing furan dicarboxylic acid from HMF, typically using noble metal-based catalysts to achieve this catalytic oxidation process. Noble metals reported to have good catalytic efficiency comprise platinum, palladium, gold, and ruthenium. Considering the cost of catalysts, developing inexpensive metal catalytic systems to achieve the conversion from HMF to FDCA has greater industrial value.

Given the important role and applications of 2,5-furan dicarboxylic acid, research on inexpensive metal catalyst systems for oxidizing HMF to FDCA is of significant importance. However, the preparation process still faces issues such as harsh reaction conditions, expensive catalysts, complex and uncontrollable catalyst preparation processes, which are unfavorable for large-scale industrial production.

### SUMMARY

In view of this, to solve the above technical problems, the present application provides a method and apparatus for preparing furan dicarboxylic acid. The catalyst and raw materials used in the preparation method provided by the present application are both inexpensive, greatly reducing the reaction cost. Moreover, the process is simple, making it an economical, environmentally friendly preparation method suitable for scaled industrial production.

To achieve the above technical objectives and effects, the present application is implemented through the following technical solutions:
The first aspect of the embodiment of the present application provides a method for preparing furan dicarboxylic acid, which comprises the following steps:
Using 5-hydroxymethylfurfural as raw material and water as reaction solvent, dissolving 5-hydroxymethylfurfural and alkali in water to obtain a 5-hydroxymethylfurfural mixed reaction solution, employing a fixed-bed reactor, using a multi-metal catalyst, loading the catalyst into a bed layer of the fixed-bed reactor, starting the fixed-bed reactor, setting the reaction temperature and reaction pressure of the fixed-bed reactor, and introducing gas into the fixed-bed reactor while pumping pure water into the fixed-bed reactor, and when the reaction temperature and reaction pressure in the fixed-bed reactor reach the set values, pumping the 5-hydroxymethylfurfural mixed reaction solution into the fixed-bed reactor, allowing the 5-hydroxymethylfurfural mixed reaction solution to first flow through the bed layer of the fixed-bed reactor loaded with catalyst to obtain an aqueous solution containing furan dicarboxylate, then precipitating solid matter through acidification, and finally obtaining furan dicarboxylic acid by filtering the solid matter.

The catalyst is a multi-metal catalyst, denoted as LaₓCo_{y}MnV_{z}.

Wherein, in the multi-metal catalyst, the V/Mn molar ratio is 1~0.5, the Co/Mn molar ratio is 1~0.5, and the La/Mn molar ratio is 0.05, 0.1, 0.15, or 0.2.

The alkali is any one or more of sodium hydroxide, potassium hydroxide, sodium carbonate, potassium carbonate, sodium bicarbonate, and potassium bicarbonate mixed in any proportion. Preferably, the alkali is at least one of sodium carbonate, potassium carbonate, sodium bicarbonate, and potassium bicarbonate.

In one embodiment, in the 5-hydroxymethylfurfural mixed reaction solution, the mass concentration of 5-hydroxymethylfurfural is 1-10%.

The molar ratio of the alkali to 5-hydroxymethylfurfural is 4:1~2:1. Preferably, the molar ratio of the alkali to 5-hydroxymethylfurfural is 3:1~2:1.

In one embodiment, the preparation process of the multi-metal catalyst is as follows:
S1: dissolving manganese source, vanadium source, cobalt source and lanthanum source in ethanol, and preparing solution A by ultrasonic dissolution;
S2: dissolving citric acid in ethanol, and preparing solution B by ultrasonic dissolution;
S3: vigorously stirring solution B, and during the vigorous stirring process, pouring solution A into solution B, stirring until a viscous gel is formed, then placing at room temperature for 24 h, then drying in a vacuum drying box at 60 °C until constant weight, taking out and grinding into powder to prepare the multi-metal catalyst precursor, transferring the multi-metal catalyst to a muffle furnace, calcining at 400 °C for 5 h, then cooling, pressing into tablets and sieving to prepare the multi-metal catalyst.

In one embodiment, in step S1, the manganese source is any one or more of manganese acetate and manganese chloride mixed in any proportion;
In step S1, the vanadium source is any one or more of sodium vanadate and vanadium acetylacetonate mixed in any proportion;
In step S1, the cobalt source is any one or more of cobalt nitrate and cobalt acetate mixed in any proportion;
In step S1, the lanthanum source is any one or more of lanthanum nitrate and lanthanum acetate mixed in any proportion;
In step S1, the molar ratio of vanadium element in the vanadium source to manganese element in the manganese source is (0.5-1):1;
In step S1, the molar ratio of cobalt element in the cobalt source to manganese element in the manganese source is (0.5-1):1;
In step S1, the molar ratio of lanthanum element in the lanthanum source to manganese element in the manganese source is selected from 0.05:1, 0.1:1, 0.15:1 or 0.2:1;
In step S3, the molar ratio of manganese element in the manganese source, vanadium element in the vanadium source, cobalt element in the cobalt source, and lanthanum element in the lanthanum source in solution A to citric acid in solution B is (Mn+V+Co+La): citric acid=1:1.5.

In one embodiment, the multi-metal catalyst has a size of 10-300 mesh, preferably, the multi-metal catalyst has a size of 30-200 mesh.

In one embodiment, when the catalyst is loaded into the bed layer of the fixed-bed reactor, the loading amount of the catalyst is 100%.

In one embodiment, the set value of the reaction temperature is 120-160 °C, and the set value of the reaction pressure is 0.5-4 MPa. Preferably, the set value of the reaction temperature is 120-140°C, and the set value of the reaction pressure is 2-3 MPa;
The gas is air or oxygen.

In one embodiment, during the process of pumping the 5-hydroxymethylfurfural mixed reaction solution into the fixed-bed reactor, the flow rate of the 5-hydroxymethylfurfural mixed reaction solution is 2-20mL/min. Preferably, during the process of pumping the 5-hydroxymethylfurfural mixed reaction solution into the fixed-bed reactor, the flow rate of the 5-hydroxymethylfurfural mixed reaction solution is 2-10mL/min.

In one embodiment, the specific process of acidification is as follows: adding an acidifying agent to the collected aqueous solution containing furan dicarboxylate, acidifying until the pH of the aqueous solution containing furan dicarboxylate is less than 1, after which a solid precipitates.

Wherein, the acidifying agent is any one or more of hydrochloric acid, sulfuric acid, nitric acid mixed in any proportion. Preferably, the acidifying agent is hydrochloric acid.

The embodiment of the second aspect of the present application provides a preparation apparatus for a method of preparing furan dicarboxylic acid, comprising a feeding system, a reaction system and a product processing system, wherein an inlet and an outlet of the reaction system are respectively connected to the feeding system and the product processing system.

The feeding system comprises a gas source and a metering pump.

The reaction system comprises a fixed-bed reactor and a reactor furnace that are interconnected, wherein a gas outlet of the gas source and an outlet of the metering pump are both connected to the fixed-bed reactor.

The product processing system comprises a cooler, a gas-liquid separator, and a storage tank.

The gas source comprises a nitrogen gas source and an oxygen/air gas source, the gas outlets of both the nitrogen gas source and the oxygen/air gas source are connected to the fixed-bed reactor through gas outlet pipes, there are two gas mass flow controllers, and the two gas mass flow controllers are respectively arranged on the gas outlet pipes.

The inlet of the metering pump is connected to a feed tank, a balance is arranged at the bottom of the feed tank, a preheater is arranged on the outside of the outlet of the metering pump, and a preheating furnace is arranged on the outside of the preheater.

A thermal insulation sleeve is arranged on an end outside the outlet of the metering pump close to the fixed-bed reactor, and the thermal insulation sleeve is located between the preheater and the fixed-bed reactor.

The gas outlet pipe passes through the preheater and the thermal insulation sleeve in sequence to connect with the fixed-bed reactor.

An outlet pipe is arranged at the outlet of the reactor furnace, the outlet of the fixed-bed reactor is connected to the outlet pipe, the outlet pipe passes through the cooler and connects to the gas-liquid separator, and the outlet of the gas-liquid separator is connected to the storage tank.

A sampling valve is arranged on the gas-liquid separator.

Compared with the existing technology, the beneficial effects of the present application are:
The present application obtains a 5-hydroxymethylfurfural mixed reaction solution by dissolving 5-hydroxymethylfurfural and alkali in water, uses a fixed bed as the reactor, employs a multi-metal LaₓCo_{y}MnV_{z} as the catalyst, and conducts the reaction at a certain temperature, oxygen pressure, and flow rate to obtain an aqueous solution of furan dicarboxylate, which is then acidified to precipitate furan dicarboxylic acid, and filtered to obtain the solid product. This method offers convenient product separation, a simple process, and has potential application value.

The present application provides a preparation device for a method of preparing furan dicarboxylic acid, where the feed tank can be used to store liquid raw materials; the metering pump can achieve continuous feeding of 5-hydroxymethylfurfural mixed reaction solution; the preheater can preheat and mix the 5-hydroxymethylfurfural mixed reaction solution; The fixed-bed reactor can be filled with catalyst and serves as the site for oxidation reactions; the reactor furnace can provide constant-temperature heat transfer medium for the reactor's heat exchange jacket; the cooler can achieve cooling and condensation of the aqueous solution containing furan dicarboxylate; the gas-liquid separator can separate gas and liquid products based on different boiling points; the storage tank can collect liquid reaction products.

In the present application, the LaₓCo_{y}MnV_{z} catalyst for catalyzing the preparation of FDCA from 5-HMF has high catalytic activity and selectivity. The presence of V in the multi-metal catalyst causes Mn to generate more oxygen vacancies, producing more active species. The presence of La promotes the interaction between Mn and V, while the introduction of Co further enhances the oxidation capability of the catalyst.

### BRIEF DESCRIPTION OF THE DRAWINGS

The Figures. described herein are provided to offer further understanding of the present application and constitute a part of the present application. The illustrative embodiments and their descriptions are used to explain the invention and do not constitute improper limitations on the invention. In the Figures:

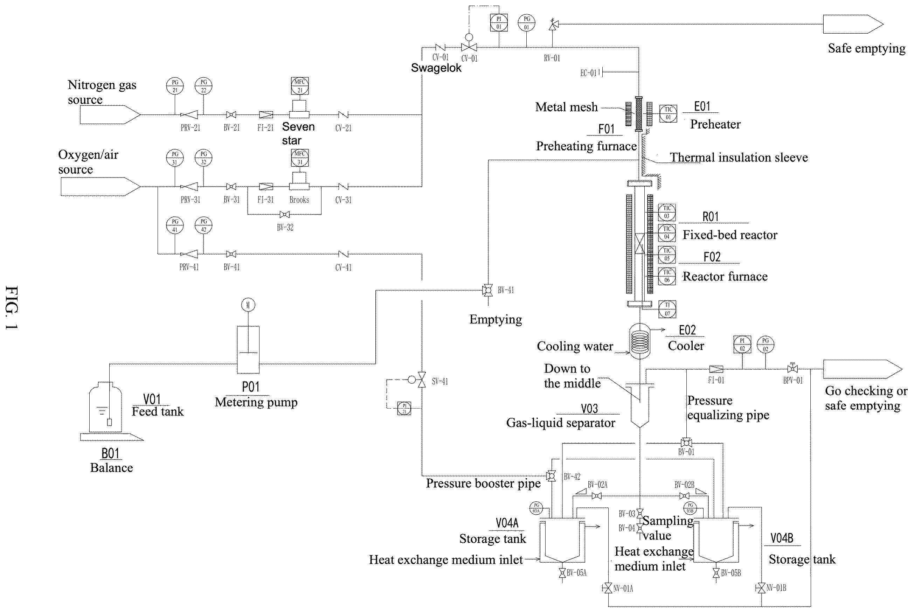

FIG. 1 is a schematic diagram of the preparation apparatus for the method of preparing furan dicarboxylic acid provided in an embodiment of the present application;
FIG. 2 is an XPS diagram of the La_{0.05}CoMnV catalyst, indicating that the prepared multi-metal catalyst La_{0.05}CoMnV contains La, Co, Mn, and V elements;
FIG. 3 is an XRD diagram of the multi-metal catalysts La_{0.05}CoMnV, La_{0.1}CoMnV, La_{0.15}CoMnV;
FIG. 4 is a Fourier Transform Infrared Spectroscopy (FTIR) analysis diagram of the multi-metal catalyst La_{0.05}CoMnV;
FIG. 5 is the Fourier transform infrared spectroscopy (FTIR) analysis diagram of the multi-metal catalyst La_{0.1}CoMnV;
FIG. 6 is the Fourier transform infrared spectroscopy (FTIR) analysis diagram of the multi-metal catalyst La_{0.15}CoMnV;
FIG. 7 is the Raman spectroscopy diagram of the multi-metal catalyst La_{0.05}CoMnV;
FIG. 8 is the BET diagram of the multi-metal catalyst La_{0.15}CoMnV.

### DETAILED DESCRIPTION

The following will combine the drawings in the embodiments of the present application to clearly and completely describe the technical solutions in the embodiments of the present application. Obviously, the described embodiments are only a part of the embodiments of the present application, not all of the embodiments. Based on the embodiments in the present application, all other embodiments obtained by those skilled in the art without creative effort fall within the scope of protection of the present application.

In the description of the present application, it should be understood that terms such as "opening", "upper", "lower", "thickness", "top", "middle", "length", "inner", "surrounding" *etc.,* which indicate orientation or positional relationships, are merely for convenience of description and simplification of the invention, and do not indicate or imply that the referenced components or elements must have a specific orientation, be constructed or operated in a specific orientation. Therefore, they should not be construed as limitations on the present application.

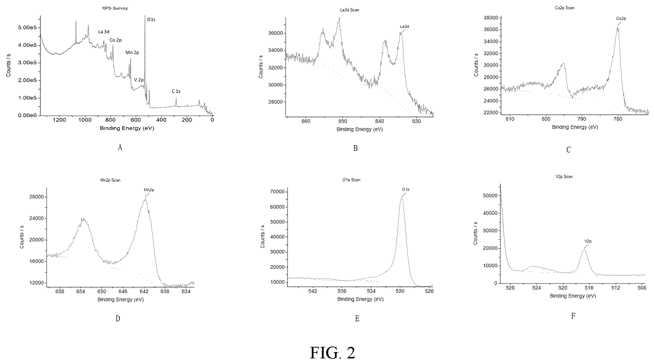

The first aspect of the embodiment of the present application provides a method for preparing furan dicarboxylic acid, which comprises the following steps:
S1: using 5-hydroxymethylfurfural as raw material and water as reaction solvent, obtaining a 5-hydroxymethylfurfural mixed reaction solution by dissolving 5-hydroxymethylfurfural and alkali in water;
wherein, in the 5-hydroxymethylfurfural mixed reaction solution, the mass concentration of 5-hydroxymethylfurfural is 1-10%;
the alkali is any one or multiple of sodium hydroxide, potassium hydroxide, sodium carbonate, potassium carbonate, sodium bicarbonate and potassium bicarbonate mixed in any proportion;
the molar ratio of alkali to 5-hydroxymethylfurfural is 4:1~2:1;
S2: employing a fixed-bed reactor as the reaction vessel, using multi-metal LaₓCo_{y}MnV_{z} as the catalyst, the catalyst is loaded into the bed layer of the fixed-bed reactor, where the loading amount of the catalyst is 100%;
in the multi-metal catalyst, the V/Mn molar ratio is 1~0.5, the Co/Mn molar ratio is 1~0.5, and the La/Mn molar ratio is 0.05, 0.1, 0.15 or 0.2;
the preparation process of the multi-metal catalyst is as follows:
   S21: dissolving the manganese source, vanadium source, cobalt source and lanthanum source in ethanol, and preparing solution A by ultrasonic dissolution; the manganese source is any one or more of manganese acetate and manganese chloride mixed in any proportion; the vanadium source is any one or more of sodium vanadate and vanadium acetylacetonate mixed in any proportion; the cobalt source is any one or more of cobalt nitrate and cobalt acetate mixed in any proportion; the lanthanum source is any one or more of lanthanum nitrate and lanthanum acetate mixed in any proportion;
   in step S1, the molar ratio of vanadium element in the vanadium source to manganese element in the manganese source is (0.5-1):1;
   the molar ratio of cobalt element in the cobalt source to manganese element in the manganese source is (0.5-1):1;
   the molar ratio of lanthanum element in the lanthanum source to manganese element in the manganese source is selected from 0.05:1, 0.1:1, 0.15:1 or 0.2:1;
   S22: dissolving citric acid in ethanol, and preparing solution B by ultrasonic dissolution;
   S23: vigorously stirring solution B, and during the vigorous stirring process, pouring solution A into solution B, stirring until a viscous gel is formed, then placing at room temperature for 24 h, then drying in a vacuum drying box at 60 °C until constant weight, taking out and grinding into powder to prepare the multi-metal catalyst precursor, transferring the multi-metal catalyst to a muffle furnace, calcining at 400°C for 5 h, then cooling, pressing into tablets and sieving to prepare the multi-metal catalyst; The prepared multi-metal catalyst has a size of 10-300 mesh, preferably 30-200 mesh;
   wherein, in step S3, the molar ratio of manganese element in the manganese source, vanadium element in the vanadium source, cobalt element in the cobalt source, and lanthanum element in the lanthanum source in solution A to citric acid in solution B is (Mn+V+Co+La): citric acid=1:1.5;
   S3: starting the fixed-bed reactor, presetting the reaction temperature inside the fixed-bed reactor to 120-160 °C and the reaction pressure to 0.5-4 MPa, and introducing air or oxygen into the fixed-bed reactor while pumping pure water into the fixed-bed reactor; when the reaction temperature and reaction pressure inside the fixed-bed reactor reach the set values, pumping the 5-hydroxymethylfurfural mixture reaction solution into the fixed-bed reactor at a flow rate of 2-20 mL/min; the 5-hydroxymethylfurfural mixture reaction solution first flows through the bed layer of the fixed-bed reactor packed with catalyst to obtain an aqueous solution containing furan dicarboxylate;
   S4: then acidifying the aqueous solution containing furan dicarboxylate to precipitate solid matter, and filtering the solid matter to obtain furan dicarboxylic acid; wherein, the specific acidification process is as follows: adding an acidifying agent to the collected aqueous solution containing furan dicarboxylate, and acidifying until the pH of the aqueous solution containing furan dicarboxylate is less than 1, after which a solid precipitates;
   the acidifying agent is any one or more of hydrochloric acid, sulfuric acid, nitric acid mixed in any proportion.
   As shown in FIG. 2, an embodiment of the second aspect of the present application provides an apparatus for preparing furan dicarboxylic acid, characterized in that comprising a feeding system, a reaction system, and a product processing system, wherein an inlet and an outlet of the reaction system are respectively connected to the feeding system and the product processing system;
   the feeding system comprises a gas source and a metering pump;
   the reaction system comprises a fixed-bed reactor and a reactor furnace that are interconnected, wherein a gas outlet of the gas source and an outlet of the metering pump are both connected to the fixed-bed reactor;
   the product processing system comprises a cooler, a gas-liquid separator, and a storage tank;
   the gas source comprises a nitrogen gas source and an oxygen/air gas source, the gas outlets of both the nitrogen gas source and the oxygen/air gas source are connected to the fixed-bed reactor through gas outlet pipes, there are two gas mass flow controllers, and the two gas mass flow controllers are respectively arranged on the gas outlet pipes;
   the inlet of the metering pump is connected to a feed tank, a balance is arranged at the bottom of the feed tank, a preheater is arranged on the outside of the outlet of the metering pump, and a preheating furnace is arranged on the outside of the preheater;
   a thermal insulation sleeve is arranged on an end outside the outlet of the metering pump close to the fixed-bed reactor, and the thermal insulation sleeve is located between the preheater and the fixed-bed reactor;
   the gas outlet pipe passes through the preheater and the thermal insulation sleeve in sequence to connect with the fixed-bed reactor;
   an outlet pipe is arranged at the outlet of the reactor furnace, the outlet of the fixed-bed reactor is connected to the outlet pipe, the outlet pipe passes through the cooler and connects to the gas-liquid separator, and the outlet of the gas-liquid separator is connected to the storage tank;
   a sampling valve is arranged on the gas-liquid separator.
   The specific process for preparing furan dicarboxylic acid using this preparation device is as follows: The prepared 5-hydroxymethylfurfural mixed reaction solution is added to the feed tank, then delivered by the metering pump at a certain flow rate through the preheater for preheating before entering the fixed-bed reactor for heated reaction. After the reaction is completed, the resulting product is cooled by the cooler, separated by the gas-liquid separator, and the reaction solution is collected in the storage tank. After being discharged through the sampling valve, an aqueous solution containing furan dicarboxylate is obtained. This aqueous solution containing furan dicarboxylate is then acidified to precipitate solid matter, and the solid matter is finally filtered to obtain furan dicarboxylic acid.

The following are specific embodiments. Unless otherwise specified, all reagents used in the embodiments of the present application can be obtained commercially.

### Embodiment 1

This embodiment provides the preparation of a multi-metal catalyst, with the specific preparation process as follows:
Preparation of solution A: Dissolve 0.02 mol of manganese acetate (3.46 g), 0.02 mol of sodium vanadate (3.68 g), 0.02 mol of cobalt nitrate hexahydrate (5.82 g), and 0.001 mol of lanthanum nitrate (0.33 g) in 200 mL of ethanol using ultrasonication;
Preparation of solution B: Dissolve citric acid (the molar ratio of manganese source, vanadium source, cobalt source, and lanthanum source in solution A to citric acid in solution B is (Mn+V+Co+La): citric acid=1.5:1, i.e., citric acid is 0.0915 mol, 17.56 g) in 200 mL of ethanol using ultrasonication;
Preparation of multi-metal catalyst: Under vigorous stirring, solution A is rapidly added to solution B, stirred until a viscous gel forms, and then allowed to fully react and age at room temperature for 24 h. Subsequently, it is dried in a vacuum drying oven at 60 °C until constant weight, then removed and ground into powder to obtain the multi-metal catalyst precursor. The multi-metal catalyst precursor is transferred to a muffle furnace and calcined in air at 400 °C for 5 h, with a heating rate of 2 °C/minute from 30 °C. Finally, the multi-metal catalyst La_{0.05}CoMnV is obtained, which is then pressed into tablets and sieved to obtain multi-metal catalysts of different mesh sizes, labeled as La_{0.05}CoMnV.

The multi-metal catalyst prepared in this embodiment is shown in Table 1.

### Embodiment 2

This embodiment provides the preparation of a multi-metal catalyst, with the specific preparation process referring to Embodiment 1. The difference from Embodiment 1 is that the amount of lanthanum nitrate used is 0.66 g (0.002 mol), and the amount of citric acid is 17.86 g. The prepared multi-metal catalyst is labeled as La_{0.1}MnCoV.

The multi-metal catalyst prepared in this embodiment is shown in Table 1.

### Embodiment 3

This embodiment provides the preparation of a multi-metal catalyst, with the specific preparation process referring to Embodiment 1. The difference from Embodiment 1 is that the amount of cobalt nitrate hexahydrate used is 2.91 g (0.01 mol), and the amount of citric acid is 14.69 g. The prepared multi-metal catalyst is labeled as La_{0.05}Co_{0.5}MnV.

The multi-metal catalyst prepared in this embodiment is shown in Table 1.

### Embodiment 4

This embodiment provides the preparation of a multi-metal catalyst, with the specific preparation process referring to Embodiment 1. The difference from Embodiment 1 is that the amount of cobalt nitrate hexahydrate used is 2.91 g (0.01 mol), the amount of sodium vanadate is 1.84 g, and the amount of citric acid is 11.8 g. The prepared multi-metal catalyst is labeled as La_{0.05}Co_{0.5}MnV_{0.5}.

The multi-metal catalyst prepared in this embodiment is shown in Table 1.

### Embodiment 5

This embodiment provides the preparation of a multi-metal catalyst, with the specific preparation process referring to Embodiment 1. The difference from Embodiment 1 is that the amount of lanthanum nitrate used is 0.99 g (0.003 mol), and the amount of citric acid is 18.16 g. The prepared multi-metal catalyst is labeled as La_{0.15}CoMnV.

The multi-metal catalyst prepared in this embodiment is shown in Table 1.

### Embodiment 6

This embodiment provides the preparation of a multi-metal catalyst, with the specific preparation process referring to Embodiment 1. The difference from Embodiment 1 is that the amount of lanthanum nitrate used is 1.32 g (0.004 mol), the amount of cobalt nitrate used is 1.83 g (0.01 mol), and the amount of citric acid used is 15.56 g. The prepared multi-metal catalyst is labeled as La_{0.2}Co_{0.5}MnV.

The multi-metal catalyst prepared in this embodiment is shown in Table 1.

### Embodiment 7

This embodiment provides the preparation of a multi-metal catalyst, with the specific preparation process referring to Embodiment 1. The difference from Embodiment 1 is that the amount of lanthanum nitrate used is 0.66 g (0.002 mol), the amount of cobalt nitrate used is 1.83 g (0.01 mol), and the amount of citric acid used is 14.99 g. The prepared multi-metal catalyst is labeled as La_{0.1}Co_{0.5}MnV.

The multi-metal catalyst prepared in this embodiment is shown in Table 1.

### Embodiment 8

This embodiment provides the preparation of a multi-metal catalyst, with the specific preparation process referring to Embodiment 1. The difference from Embodiment 1 is that the amount of lanthanum nitrate used is 1.32 g (0.004 mol), the amount of citric acid is 18.44 g, and the prepared multi-metal catalyst is labeled as La_{0.2}CoMnV.

The multi-metal catalyst prepared in this embodiment is shown in Table 1.

### Embodiment 9

This embodiment provides the preparation of a multi-metal catalyst, with the specific preparation process referring to Embodiment 1. The difference from Embodiment 1 is that the amount of cobalt nitrate hexahydrate used is 4.37 g (0.015 mol), the amount of sodium vanadate is 2.76 g (0.015 mol), the amount of citric acid is 14.70 g, and the prepared multi-metal catalyst is labeled as La_{0.05}Co_{0.75}MnV_{0.75}.

The multi-metal catalyst prepared in this embodiment is shown in Table 1.

**Table 1 Multi-metal catalyst**

| Item | multi-metal catalyst | Mangan ese acetate/mol | Sodium vanadate/mol | Cobalt nitrate/mol | Lanthan um nitrate/mol | (Mn+V+Co+ La)/mol | Citric acid/mol |
|---|---|---|---|---|---|---|---|
| Embodiment 1 | La_{0.05}CoMnV | 0.02 | 0.02 | 0.02 | 0.001 | 0.061 | 0.0915 |
| Embodiment 2 | La_{0.1}MnCoV | 0.02 | 0.02 | 0.02 | 0.002 | 0.062 | 0.093 |
| Embodiment 3 | La_{0.05}Co_{0.5}Mn V | 0.02 | 0.02 | 0.01 | 0.001 | 0.051 | 0.0765 |
| Embodiment 4 | La_{0.05}Co_{0.5}Mn V_{0.5} | 0.02 | 0.01 | 0.01 | 0.001 | 0.041 | 0.0615 |
| Embodiment 5 | La_{0.15}CoMnV | 0.02 | 0.02 | 0.02 | 0.003 | 0.063 | 0.094 |
| Embodiment 6 | La_{0.2}Co_{0.5}MnV | 0.02 | 0.02 | 0.01 | 0.004 | 0.054 | 0.081 |
| Embodiment 7 | La_{0.1}Co_{0.5}MnV | 0.02 | 0.02 | 0.01 | 0.002 | 0.052 | 0.078 |
| Embodiment 8 | La_{0.2}CoMnV | 0.02 | 0.02 | 0.02 | 0.004 | 0.064 | 0.096 |
| Embodiment 9 | La_{0.05}Co_{0.75}Mn V_{0.75} | 0.02 | 0.015 | 0.015 | 0.001 | 0.051 | 0.0765 |

### Experimental example 1

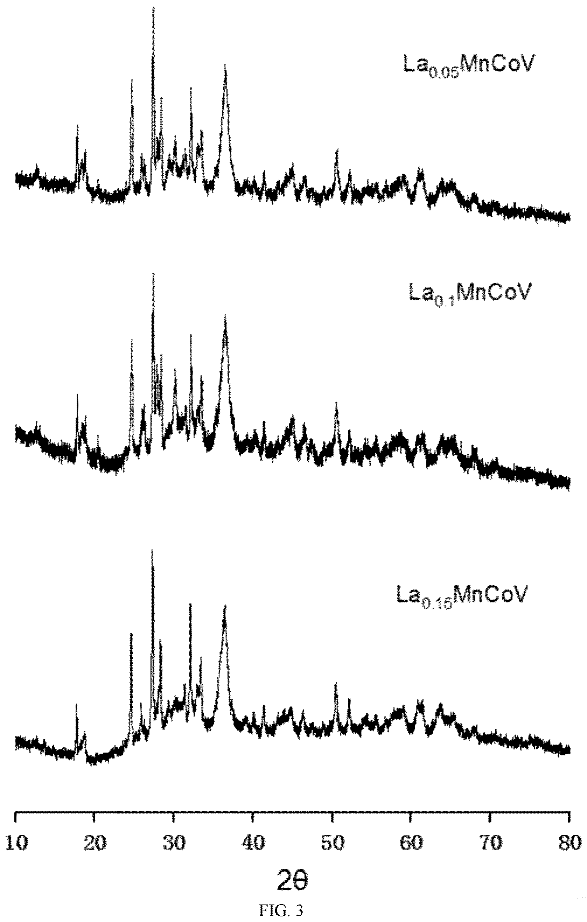

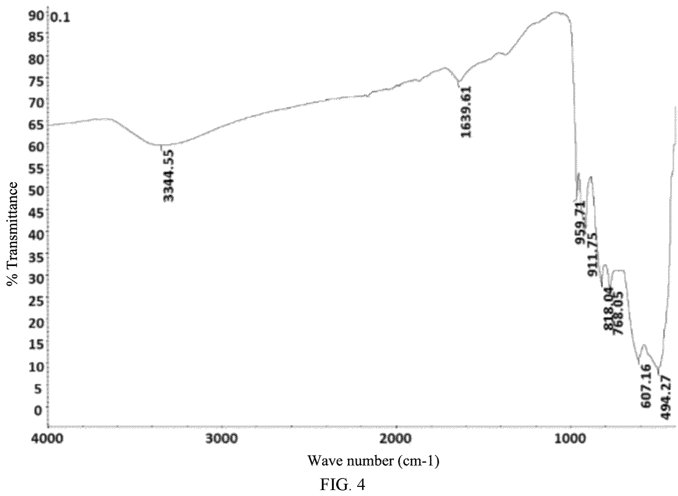

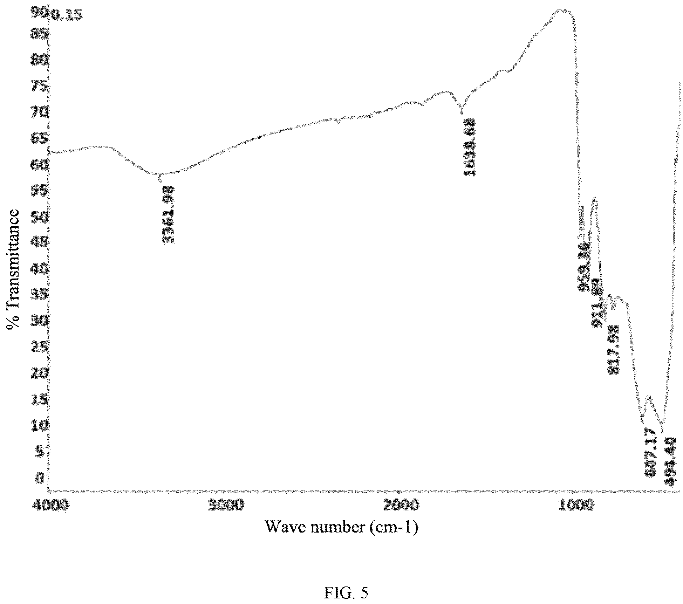

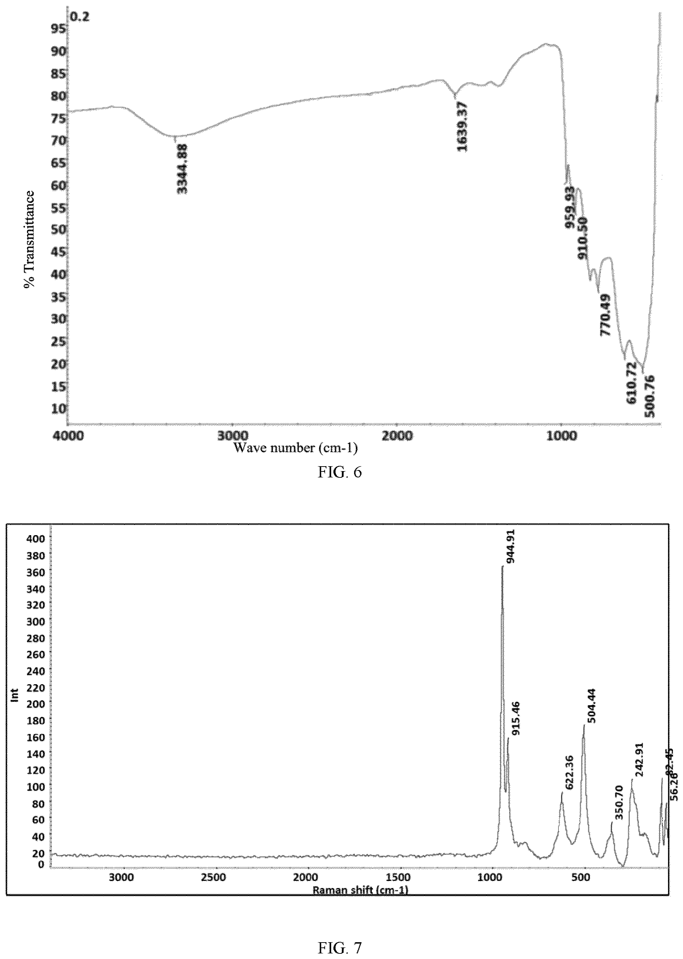

Performance tests were conducted on the multi-metal catalyst La_{0.05}CoMnV prepared in embodiment 1, the multi-metal catalyst La_{0.1}CoMnV prepared in embodiment 2, and the multi-metal catalyst La_{0.15}CoMnV prepared in embodiment 5;
The XPS spectra of multi-metal catalyst La_{0.05}CoMnV, multi-metal catalyst La_{0.1}CoMnV, and multi-metal catalyst La_{0.15}CoMnV are shown in FIG. 2A. FIG. 2A demonstrates the composition of the prepared multi-metal catalysts, namely that the multi-metal catalysts contain La, Co, Mn, and V elements; FIGS. 2B, 2C, 2D, 2E, and 2F show the characteristic photoelectron spectral lines of La, Co, Mn, O, and V, respectively, confirming the presence of La, Co, Mn, and V in the prepared multi-metal catalyst;
The XRD patterns of multi-metal catalyst La_{0.05}CoMnV, multi-metal catalyst La_{0.1}CoMnV, and multi-metal catalyst La_{0.15}CoMnV are shown in FIG. 3. From FIG. 3, it can be seen that changing the amount of La does not significantly alter the diffraction peaks in the XRD, while Mn, Co, and V form a spinel phase, which is beneficial for the valence changes of the catalyst during the oxidation process. No obvious derivative peaks of La species were observed, indicating the high dispersion of La. The presence of La promotes the interaction between Mn and V, while the introduction of Co further enhances the oxidation capability of the catalyst;
The infrared spectrum of La_{0.05}MnCoV catalyst is shown in FIG. 4, the infrared spectrum of La_{0.1}MnCoV catalyst is shown in FIG. 5, and the infrared spectrum of La_{0.15}MnCoV catalyst is shown in FIG. 6. Infrared characterization analysis reveals that changing the proportion of La does not significantly alter the structural characteristics of the catalyst;
The Raman spectroscopy of the multi-metal catalyst La_{0.05}CoMnV is shown in FIG. 7. FIG. 7 shows the stretching vibrations of CoMnVOₓ tetrahedral structure at 500~900 cm⁻¹ and the bending vibrations of the tetrahedral structure at 200~500 cm⁻¹. Below 200 cm⁻¹ are the rigid motions of Mn-O polyhedra and VO₄ tetrahedra. The peak at 622 cm⁻¹ is attributed to the symmetric stretching vibration of (Co/Mn)O₆ octahedra, while the peak at 242 cm⁻¹ is the antisymmetric stretching vibration of (Co/Mn)O₆ octahedra. Raman peaks above 900 cm⁻¹ may be due to some distortions in the crystal lattice or related to stretching overtones.

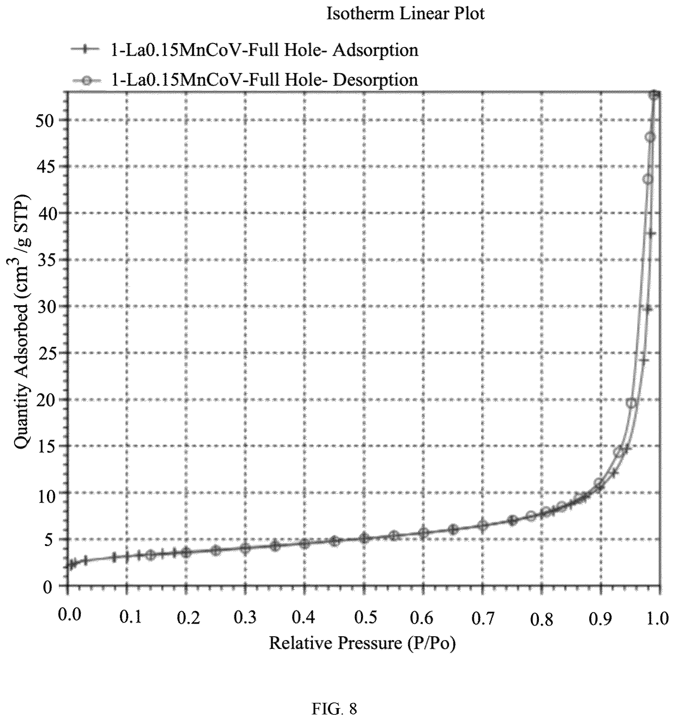

The BET diagram of the multi-metal catalyst La_{0.15}CoMnV is shown in FIG. 8. This catalyst has a surface area of 12.8 m²/g and an adsorption average pore diameter of 25.43117 nm. From the BET results, it can be observed that the catalyst exhibits a distinct mesoporous structure.

In summary, the presence of V in the prepared LaₓCo_{y}MnV_{z} catalyst causes Mn to produce more oxygen vacancies, generating more active species. The presence of La promotes the interaction between Mn and V, while the introduction of Co further enhances the oxidation capacity of the catalyst.

### Embodiment 10

This embodiment provides a method for preparing furan dicarboxylic acid, which comprises the following steps:
Dissolving 5-hydroxymethylfurfural, i.e., HMF (63 g, 0.5 mol) in 2000 g of water and add sodium hydroxide (40 g, 1 mol), where the molar ratio of sodium hydroxide (base) to 5-hydroxymethylfurfural is 2:1, and thoroughly stirring to prepare a 5-hydroxymethylfurfural mixed reaction solution. Loading the 100 mesh multi-metal catalyst La_{0.05}Co_{0.5}MnV prepared in Embodiment 3 into the bed layer of the fixed-bed reactor (volume 40 mL). Setting the reaction temperature to 130°C and reaction pressure to 2 MPa, starting the metering pump of the fixed-bed reactor, introducing oxygen into the fixed-bed reactor, and pumping pure water at a rate of 20 mL/min. After the reaction parameters reach the set values, pumping the 5-hydroxymethylfurfural mixed reaction solution at a rate of 2 mL/min. After the reaction is completed, collecting the reaction liquid and taking samples for detection. Detection conditions: Hitachi L2000 HPLC System, Alltech C18 column; Mobile phase methanol: 0.5 wt.% trifluoroacetic acid aqueous solution 20:80; flow rate: 1.0 mL/min; column temperature: 30°C; Detector: DAD, detection wavelength: 264 nm, the measured purity of furan dicarboxylic acid (FDCA) was 99.3%. The collected reaction solution was acidified with hydrochloric acid to pH < 1, resulting in precipitation of solids, which were filtered and dried to obtain FDCA with a yield of 84%.

The purity and yield of the prepared FDCA are shown in Table 2.

### Embodiment 11

This embodiment provides a method for preparing furan dicarboxylic acid, which differs from Embodiment 10 in that the mass of water in this embodiment is 1157 g, i.e., the HMF mass concentration is changed to 5%, with the remaining operational steps being the same as in Embodiment 10.

Test results show: The yield of FDCA was 76%, with a purity of 98.3%.

The purity and yield of the prepared FDCA are shown in Table 2.

### Embodiment 12

This embodiment provides a method for preparing furan dicarboxylic acid, which differs from embodiment 10 in that the mass of sodium hydroxide in this embodiment is 80 g (2.0 mol), i.e., the molar ratio of alkali to 5-hydroxymethylfurfural is 4: 1, with the remaining operational steps being the same as embodiment 10.

Test results show: The yield of FDCA was 81%, with a purity of 99.0%.

The purity and yield of the prepared FDCA are shown in Table 2.

### Embodiment 13

This embodiment provides a method for preparing furan dicarboxylic acid, which differs from embodiment 10 in that the mass of water in this embodiment is 684.5 g, i.e., the mass concentration of HMF is changed to 8%, with the remaining operational steps being the same as embodiment 10.

Test results show: FDCA yield is 75%, with a purity of 98.8%.

The purity and yield of the prepared FDCA are shown in Table 2.

### Embodiment 14

This embodiment provides a method for preparing furan dicarboxylic acid, which differs from embodiment 10 in that the water mass in this embodiment is 527 g, meaning the mass concentration of 5-hydroxymethylfurfural is 10%, with the remaining operational steps the same as embodiment 10.

Test results show: FDCA yield is 72%, with a purity of 98.2%.

The purity and yield of the prepared FDCA are shown in Table 2.

### Embodiment 15

This embodiment provides a method for preparing furan dicarboxylic acid, which differs from embodiment 10 in that the amount of water added in this embodiment is 6197 g, meaning the mass concentration of 5-hydroxymethylfurfural in the mixed reaction solution is 1%, with the remaining operational steps the same as embodiment 10.

Test results show: FDCA yield is 70%, with a purity of 99.8%.

The purity and yield of the prepared FDCA are shown in Table 2.

### Embodiment 16

This embodiment provides a method for preparing furan dicarboxylic acid, which differs from embodiment 10 in that the mass of sodium hydroxide in this embodiment is 60 g (1.5 mol), meaning the molar ratio of alkali to 5-hydroxymethylfurfural is 3:1, with the remaining operational steps the same as embodiment 10.

Test results show: FDCA yield is 80%, with a purity of 99.6%.

The purity and yield of the prepared FDCA are shown in Table 2.

**Table 2**

| Item | HMF addition amount/ g | Alkali addition amount/ g | Water addition amount/ g | Mola r ratio of alkali to HMF | Mass concentration of 5-hydroxymethylfurfural /% | FDCA purity/ % | FDCA yield/ % |
|---|---|---|---|---|---|---|---|
| Embodime nt 10 | 63 | 40 | 2000 | 2: 1 | 3.0 | 99.3 | 84 |
| Embodime nt 11 | 63 | 40 | 1157 | 2: 1 | 5 | 98.3 | 76 |
| Embodime nt 12 | 63 | 80 | 2000 | 4: 1 | 2.94 | 99.0 | 81 |
| Embodime nt 13 | 63 | 40 | 684.5 | 2: 1 | 8 | 98.8 | 75 |
| Embodime nt 14 | 63 | 40 | 527 | 2: 1 | 10 | 98.2 | 72 |
| Embodime nt 15 | 63 | 40 | 6197 | 2: 1 | 1 | 99.8 | 70 |
| Embodime nt 16 | 63 | 60 | 2000 | 3: 1 | 2.97 | 99.6 | 80 |

### Embodiment 17

This embodiment provides a method for preparing furan dicarboxylic acid, which differs from embodiment 10 in that the 100 mesh multi-metal catalyst La_{0.05}Co_{0.5}MnV is replaced with 200 mesh in this embodiment, while the remaining operational steps are the same as embodiment 10.

Test results show: the yield of FDCA is 91%, with a purity of 99.4%.

The specific purity and yield of the prepared FDCA are shown in Table 3.

### Embodiment 18

This embodiment provides a method for preparing furan dicarboxylic acid, which differs from embodiment 10 in that the 100 mesh multi-metal catalyst La_{0.05}Co_{0.5}MnV is replaced with 30 mesh in this embodiment, while the remaining operational steps are the same as embodiment 10.

Test results show: FDCA yield is 82%, with a purity of 98.1%.

The specific purity and yield of the prepared FDCA are shown in Table 3.

### Embodiment 19

This embodiment provides a method for preparing furan dicarboxylic acid, which differs from embodiment 10 in that this embodiment replaces the 100 mesh multi-metal catalyst La_{0.05}Co_{0.5}MnV with 50 mesh, while the remaining operational steps are the same as embodiment 10.

Test results show: FDCA yield is 77%, with a purity of 97.6%.

The specific purity and yield of the prepared FDCA are shown in Table 3.

### Embodiment 20

This embodiment provides a method for preparing furan dicarboxylic acid, which differs from embodiment 10 in that this embodiment replaces the multi-metal catalyst La_{0.05}Co_{0.5}MnV prepared in embodiment 3 with the multi-metal catalyst La_{0.05}CoMnV prepared in embodiment 1, while the remaining operational steps are the same as embodiment 10.

Test results show: FDCA yield is 79%, with a purity of 98.3%.

The specific purity and yield of the prepared FDCA are shown in Table 3.

### Embodiment 21

This embodiment provides a method for preparing furan dicarboxylic acid, which differs from Embodiment 10 in that the multi-metal catalyst La_{0.05}Co_{0.5}MnV prepared in Embodiment 3 is replaced with the multi-metal catalyst La_{0.5}sCo_{0.5}MnV_{0.5} prepared in Embodiment 4, with all other operational steps remaining the same as in Embodiment 10.

Test results show: FDCA yield is 94%, with a purity of 99.1%.

The specific purity and yield of the prepared FDCA are shown in Table 3.

### Embodiment 22

This embodiment provides a method for preparing furan dicarboxylic acid, which differs from Embodiment 10 in that the multi-metal catalyst La_{0.05}Co_{0.5}MnV prepared in Embodiment 3 is replaced with the multi-metal catalyst La_{0.2}Co_{0.5}MnV prepared in Embodiment 6, with all other operational steps remaining the same as in Embodiment 10.

Test results show: FDCA yield is 81%, with a purity of 97.9%.

The specific purity and yield of the prepared FDCA are shown in Table 3.

### Embodiment 23

This embodiment provides a method for preparing furan dicarboxylic acid, which differs from Embodiment 10 in that the multi-metal catalyst La_{0.05}Co_{0.5}MnV prepared in Embodiment 3 is replaced with the multi-metal catalyst La_{0.1}Co_{0.5}MnV prepared in Embodiment 7, with all other operational steps remaining the same as in Embodiment 10.

Test results show: FDCA yield is 87%, with a purity of 98.8%.

The specific purity and yield of the prepared FDCA are shown in Table 3.

### Embodiment 24

This embodiment provides a method for preparing furan dicarboxylic acid, which differs from Embodiment 10 in that the multi-metal catalyst La_{0.05}Co_{0.5}MnV prepared in Embodiment 3 is replaced with the multi-metal catalyst La_{0.1}MnCoV prepared in Embodiment 2, with all other operational steps remaining the same as in Embodiment 10.

Test results show: FDCA yield is 77%, with a purity of 99.3%.

The specific purity and yield of the prepared FDCA are shown in Table 3.

### Embodiment 25

This embodiment provides a method for preparing furan dicarboxylic acid, which differs from embodiment 10 in that the multi-metal catalyst La_{0.05}Co_{0.5}MnV prepared in embodiment 3 is replaced with the multi-metal catalyst La_{0.15}CoMnV prepared in embodiment 5, with all other operational steps remaining the same as embodiment 10.

Test results show: FDCA yield is 75%, with a purity of 99.5%.

The specific purity and yield of the prepared FDCA are shown in Table 3.

### Embodiment 26

This embodiment provides a method for preparing furan dicarboxylic acid, which differs from embodiment 10 in that the multi-metal catalyst La_{0.05}Co_{0.5}MnV prepared in embodiment 3 is replaced with the multi-metal catalyst La_{0.2}CoMnV prepared in embodiment 8, with all other operational steps remaining the same as embodiment 10.

Test results show: FDCA yield is 70%, with a purity of 98.6%.

The specific purity and yield of the prepared FDCA are shown in Table 3.

### Embodiment 27

This embodiment provides a method for preparing furan dicarboxylic acid, which differs from Embodiment 10 in that the multi-metal catalyst La_{0.05}Co_{0.5}MnV prepared in Embodiment 3 is replaced with the multi-metal catalyst La_{0.05}Co_{0.75}MnV_{0.75} prepared in Embodiment 9, with the remaining operational steps being the same as Embodiment 10.

Test results show: FDCA yield is 69%, with a purity of 98.2%.

The specific purity and yield of the prepared FDCA are shown in Table 3.

**Table 3**

| Item | multi-metal catalyst | Catalyst mesh size/mesh | FDCA purity/% | FDCA yield/% |
|---|---|---|---|---|
| Embodiment 10 | La_{0.05}Co_{0.5}MnV | 100 | 99.3 | 84 |
| Embodiment 17 | La_{0.05}Co_{0.5}MnV | 200 | 99.4 | 91 |
| Embodiment 18 | La_{0.05}Co_{0.5}MnV | 30 | 98.1 | 82 |
| Embodiment 19 | La_{0.05}Co_{0.5}MnV | 50 | 97.6 | 77 |
| Embodiment 20 | La_{0.05}CoMnV | 100 | 98.3 | 79 |
| Embodiment 21 | La_{0.5}Co_{0.5}MnV_{0.5} | 100 | 99.1 | 94 |
| Embodiment 22 | La_{0.2}Co_{0.5}MnV | 100 | 97.9 | 81 |
| Embodiment 23 | La_{0.1}Co_{0.5}MnV | 100 | 98.8 | 87 |
| Embodiment 24 | La_{0.1}CoMnV | 100 | 99.3 | 77 |
| Embodiment 25 | La_{0.15}CoMnV | 100 | 99.5 | 75 |
| Embodiment 26 | La_{0.2}CoMnV | 100 | 98.6 | 70 |
| Embodiment 27 | La_{0.05}Co_{0.75}MnV_{0.75} | 100 | 98.2 | 69 |

### Embodiment 28

This embodiment provides a method for preparing furan dicarboxylic acid, which differs from Embodiment 10 in that the reaction temperature of 130 °C is replaced with 120 °C in this embodiment, while the remaining operational steps are the same as Embodiment 10.

Test results show: the yield of FDCA was 76%, with a purity of 98.6%.

The specific purity and yield of the prepared FDCA are shown in Table 4.

### Embodiment 29

This embodiment provides a method for preparing furan dicarboxylic acid, which differs from Embodiment 10 in that the reaction temperature of 130 °C is replaced with 150 °C in this embodiment, while the remaining operational steps are the same as Embodiment 10.

Test results show: the yield of FDCA was 80%, with a purity of 98.4%.

The specific purity and yield of the prepared FDCA are shown in Table 4.

### Embodiment 30

This embodiment provides a method for preparing furan dicarboxylic acid, which differs from Embodiment 10 in that the reaction temperature is changed from 130 °C to 160 °C, while all other operational steps remain the same as in Embodiment 10.

Test results show: FDCA yield is 72%, with a purity of 99.1%.

The specific purity and yield of the prepared FDCA are shown in Table 4.

### Embodiment 31

This embodiment provides a method for preparing furan dicarboxylic acid, which differs from Embodiment 10 in that oxygen is replaced with air, while all other operational steps remain the same as in Embodiment 10.

Test results show: FDCA yield is 74%, with a purity of 98.9%.

The specific purity and yield of the prepared FDCA are shown in Table 4.

### Embodiment 32

This embodiment provides a method for preparing furan dicarboxylic acid, which differs from Embodiment 10 in that the reaction pressure is replaced from 2 MPa to 0.5 MPa in this embodiment, with the remaining operational steps being the same as Embodiment 10.

Test results show: The yield of FDCA is 80%, with a purity of 98.6%.

The specific purity and yield of the prepared FDCA are shown in Table 4.

### Embodiment 33

This embodiment provides a method for preparing furan dicarboxylic acid, which differs from Embodiment 10 in that the reaction pressure is replaced from 2 MPa to 3MPa in this embodiment, with the remaining operational steps being the same as Embodiment 10.

Test results show: The yield of FDCA is 88%, with a purity of 98.8%.

The specific purity and yield of the prepared FDCA are shown in Table 4.

### Embodiment 34

This embodiment provides a method for preparing furan dicarboxylic acid, which differs from Embodiment 10 in that the reaction pressure is changed from 2 MPa to 4 MPa, with all other operational steps remaining the same as in Embodiment 10.

Test results show: FDCA yield is 92%, with a purity of 99.5%.

The specific purity and yield of the prepared FDCA are shown in Table 4.

### Embodiment 35

This embodiment provides a method for preparing furan dicarboxylic acid, which differs from Embodiment 10 in that the flow rate of the 5-hydroxymethylfurfural mixed reaction solution is changed from 2 mL/min to 20 mL/min, with all other operational steps remaining the same as in Embodiment 10.

Test results show: The yield of FDCA was 71%, with a purity of 97.7%.

The specific purity and yield of the prepared FDCA are shown in Table 4.

### Embodiment 36

This embodiment provides a method for preparing furan dicarboxylic acid, which differs from embodiment 10 in that the flow rate of the 5-hydroxymethylfurfural mixture reaction solution is changed from 2 mL/min to 10 mL/min, with all other operational steps remaining the same as in embodiment 10.

Test results show: The yield of FDCA was 78%, with a purity of 98.6%.

The specific purity and yield of the prepared FDCA are shown in Table 4.

### Embodiment 37

This embodiment provides a method for preparing furan dicarboxylic acid, which differs from embodiment 10 in that the flow rate of the 5-hydroxymethylfurfural mixture reaction solution is changed from 2 mL/min to 5 mL/min, with all other operational steps remaining the same as in embodiment 10.

Test results show: FDCA yield is 89%, with a purity of 99.3%.

The specific purity and yield of the prepared FDCA are shown in Table 4.

**Table 4**

| Item | Reaction temperature /°C | Gas input | Reaction pressure/MPa | 5-hydroxymethylfurfu ral mixed reaction solution flow rate/mL/min | FDCA purity/ % | FDCA yield/ % |
|---|---|---|---|---|---|---|
| Embodimen t 10 | 130 | oxygen | 2 | 2 | 99.3 | 84 |
| Embodimen t 28 | 120 | oxygen | 2 | 2 | 98.6 | 76 |
| Embodimen t 29 | 150 | oxygen | 2 | 2 | 98.4 | 80 |
| Embodimen t 30 | 160 | oxygen | 2 | 2 | 99.1 | 72 |
| Embodimen t 31 | 130 | air | 2 | 2 | 98.9 | 74 |
| Embodimen t 32 | 130 | oxygen | 0.5 | 2 | 98.6 | 80 |
| Embodimen t 33 | 130 | oxygen | 3 | 2 | 98.8 | 88 |
| Embodimen t 34 | 130 | oxygen | 4 | 2 | 99.5 | 92 |
| Embodimen t 35 | 130 | oxygen | 2 | 20 | 97.7 | 71 |
| Embodimen t 36 | 130 | oxygen | 2 | 10 | 98.6 | 78 |
| Embodimen t 37 | 130 | oxygen | 2 | 5 | 99.3 | 89 |

Through the above embodiments, it can be found that the LaₓCo_{y}MnV_{z} catalyst for catalyzing the preparation of FDCA from 5-HMF has high catalytic activity and selectivity. By changing reaction conditions, such as reaction temperature, reaction pressure, liquid flow rate, *etc.,* the yield of FDCA can be further improved, which also provides new ideas and directions for technical personnel in this field to obtain FDCA with higher yields.

The present application obtains a 5-hydroxymethylfurfural mixed reaction solution by dissolving 5-hydroxymethylfurfural and alkali in water, uses a fixed bed as the reactor, employs a multi-metal LaₓCo_{y}MnV_{z} as the catalyst, and conducts the reaction at a certain temperature, oxygen pressure, and flow rate to obtain an aqueous solution of furan dicarboxylate, which is then acidified to precipitate furan dicarboxylic acid, and filtered to obtain the solid product. This method offers convenient product separation, a simple process, and has potential application value.

In the description of this specification, references to terms such as "an embodiment," "example," "specific example," *etc.,* indicate that the specific features, structures, materials, or characteristics described in connection with that embodiment or example are comprised in at least one embodiment or example of the invention. In this specification, the illustrative expressions of the above terms do not necessarily refer to the same embodiment or example. Moreover, the described specific features, structures, materials, or characteristics may be combined in any suitable manner in one or more embodiments or examples.

The above demonstrates and describes the basic principles, main features, and advantages of the invention. Technical personnel in this field should understand that the invention is not limited by the above embodiments; the above embodiments and descriptions in the specification only explain the principles of the invention. Without departing from the spirit and scope of the invention, the invention will have various changes and improvements, all of which fall within the scope of protection of the present application.

## Claims

1. A method for preparing furan dicarboxylic acid, **characterized in that** the method comprises the following steps:
using 5-hydroxymethylfurfural as raw material and water as reaction solvent, dissolving 5-hydroxymethylfurfural and alkali in water to obtain a 5-hydroxymethylfurfural mixed reaction solution, employing a fixed-bed reactor, using a catalyst, loading the catalyst into a bed layer of the fixed-bed reactor, starting the fixed-bed reactor, setting the reaction temperature and reaction pressure of the fixed-bed reactor, and introducing gas into the fixed-bed reactor while pumping pure water into the fixed-bed reactor, and when the reaction temperature and reaction pressure in the fixed-bed reactor reach the set values, pumping the 5-hydroxymethylfurfural mixed reaction solution into the fixed-bed reactor, allowing the 5-hydroxymethylfurfural mixed reaction solution to first flow through the bed layer of the fixed-bed reactor loaded with catalyst to obtain an aqueous solution containing furan dicarboxylate, then precipitating solid matter through acidification, and finally obtaining furan dicarboxylic acid by filtering the solid matter;
the catalyst is a multi-metal catalyst, denoted as LaₓCo_{y}MnV_{z};
wherein, in the multi-metal catalyst, the V/Mn molar ratio is 1~0.5, the Co/Mn molar ratio is 1~0.5, and the La/Mn molar ratio is 0.05, 0.1, 0.15, or 0.2;
the alkali is any one or more of sodium hydroxide, potassium hydroxide, sodium carbonate, potassium carbonate, sodium bicarbonate, and potassium bicarbonate mixed in any proportion.

2. The method for preparing furan dicarboxylic acid according to claim 1, **characterized in that**, in the 5-hydroxymethylfurfural mixed reaction solution, the mass concentration of 5-hydroxymethylfurfural is 1-10%;
the molar ratio of the alkali to 5-hydroxymethylfurfural is 4:1~2:1.

3. The method for preparing furan dicarboxylic acid according to claim 1, **characterized in that** the preparation process of the multi-metal catalyst is as follows:
S1: dissolving manganese source, vanadium source, cobalt source and lanthanum source in ethanol, and preparing solution A by ultrasonic dissolution;
S2: dissolving citric acid in ethanol, and preparing solution B by ultrasonic dissolution;
S3: vigorously stirring solution B, and during the vigorous stirring process, pouring solution A into solution B, stirring until a viscous gel is formed, then placing at room temperature for 24 h, then drying in a vacuum drying box at 60°C until constant weight, taking out and grinding into powder to prepare the multi-metal catalyst precursor, transferring the multi-metal catalyst to a muffle furnace, calcining at 400 °C for 5 h, then cooling, pressing into tablets and sieving to prepare the multi-metal catalyst.

4. The method for preparing furan dicarboxylic acid according to claim 3, **characterized in that** in step S1, the manganese source is any one or more of manganese acetate and manganese chloride mixed in any proportion;
in step S1, the vanadium source is any one or more of sodium vanadate and vanadium acetylacetonate mixed in any proportion;
in step S1, the cobalt source is any one or more of cobalt nitrate and cobalt acetate mixed in any proportion;
in step S1, the lanthanum source is any one or more of lanthanum nitrate and lanthanum acetate mixed in any proportion;
in step S1, the molar ratio of vanadium element in the vanadium source to manganese element in the manganese source is (0.5-1):1;
in step S1, the molar ratio of cobalt element in the cobalt source to manganese element in the manganese source is (0.5-1):1;
in step S1, the molar ratio of lanthanum element in the lanthanum source to manganese element in the manganese source is selected from 0.05:1, 0.1:1, 0.15:1 or 0.2:1;
in step S3, the molar ratio of manganese element in the manganese source, vanadium element in the vanadium source, cobalt element in the cobalt source, and lanthanum element in the lanthanum source in solution A to citric acid in solution B is (Mn+V+Co+La): citric acid=1:1.5.

5. The method for preparing furan dicarboxylic acid according to claim 3, **characterized in that** the multi-metal catalyst has a size of 10-300 mesh.

6. The method for preparing furan dicarboxylic acid according to claim 1, **characterized in that** when the catalyst is loaded into the bed layer of the fixed-bed reactor, the loading amount of the catalyst is 100%.

7. The method for preparing furan dicarboxylic acid according to claim 1, **characterized in that** the set value of the reaction temperature is 120-160 °C, the set value of the reaction pressure is 0.5-4MPa, and the gas is air or oxygen.

8. The method for preparing furan dicarboxylic acid according to claim 1, **characterized in that** during the process of pumping the 5-hydroxymethylfurfural mixed reaction solution into the fixed-bed reactor, the flow rate of the 5-hydroxymethylfurfural mixed reaction solution is 2-20 mL/min.

9. The method for preparing furan dicarboxylic acid according to claim 1, **characterized in that** the specific process of acidification is: adding an acidifying agent to the collected aqueous solution containing furan dicarboxylate, acidifying until the pH of the aqueous solution containing furan dicarboxylate is less than 1, after which a solid precipitates;
wherein, the acidifying agent is any one or more of hydrochloric acid, sulfuric acid, nitric acid mixed in any proportion.

10. A preparation apparatus for the method of preparing furan dicarboxylic acid according to any one of claims 1-9, **characterized in that** comprising a feeding system, a reaction system, and a product processing system, wherein an inlet and an outlet of the reaction system are respectively connected to the feeding system and the product processing system;
the feeding system comprises a gas source and a metering pump;
the reaction system comprises a fixed-bed reactor and a reactor furnace that are interconnected, wherein a gas outlet of the gas source and an outlet of the metering pump are both connected to the fixed-bed reactor;
the product processing system comprises a cooler, a gas-liquid separator, and a storage tank;
the gas source comprises a nitrogen gas source and an oxygen/air gas source, the gas outlets of both the nitrogen gas source and the oxygen/air gas source are connected to the fixed-bed reactor through gas outlet pipes, there are two gas mass flow controllers, and the two gas mass flow controllers are respectively arranged on the gas outlet pipes;
the inlet of the metering pump is connected to a feed tank, a balance is arranged at the bottom of the feed tank, a preheater is arranged on the outside of the outlet of the metering pump, and a preheating furnace is arranged on the outside of the preheater;
a thermal insulation sleeve is arranged on an end outside the outlet of the metering pump close to the fixed-bed reactor, and the thermal insulation sleeve is located between the preheater and the fixed-bed reactor;
the gas outlet pipe passes through the preheater and the thermal insulation sleeve in sequence to connect with the fixed-bed reactor;
an outlet pipe is arranged at the outlet of the reactor furnace, the outlet of the fixed-bed reactor is connected to the outlet pipe, the outlet pipe passes through the cooler and connects to the gas-liquid separator, and the outlet of the gas-liquid separator is connected to the storage tank;
a sampling valve is arranged on the gas-liquid separator.
